# FASCICULE DE BREVET EUROPEEN

(11) **EP 2 038 013 B1**
(45) Date de publication et mention de la délivrance du brevet: **06.11.2013**
(21) Numéro de dépôt: 07803824.7
(22) Date de dépôt: 02.07.2007
(51) Int. Cl.: A61Q 19/08, A61K 8/92, A61K 8/19, A61K 8/67, A61K 8/27, A61K 8/36

(54) **COMPOSITION COSMETIQUE A BASE D'ACIDE GRAS POLYINSATURES ET SES UTILISATIONS**
KOSMETISCHE ZUSAMMENSETZUNG AUF DER BASIS MEHRFACH UNGESÄTTIGTER FETTSÄUREN UND IHRE VERWENDUNG
COSMETIC COMPOSITION BASED ON POLYUNSATURATED FATTY ACIDS AND ITS USES

(30) Priorité: 30.06.2006 FR 0605953
(43) Date de publication de la demande: 25.03.2009
(73) Titulaire: LABORATOIRES M&L, 04100 Manosque (FR)
(72) Inventeur: PIERRISNARD, Jean-Louis, F-04700 Oraison (FR); MILLOU, Yves, F-04210 Valensole (FR); FONTES, Katia, F-13650 Meyrargues (FR); TOUREL, Cécile, F-13100 Aix en Provence (FR)
(74) Mandataire: Novagraaf Technologies
(86) Numéro de dépôt international: PCT/FR2007/001115
(87) Numéro de publication internationale: WO 2008/000974

(56) Documents cités:
- EP-A2- 0 279 136
- WO-A-97/46220
- WO-A-03/018730
- WO-A-2005/039727
- WO-A-2006/059078
- WO-A2-01/37792
- FR-A1- 2 606 279
- FR-A1- 2 704 390
- FR-A1- 2 842 420
- GB-A- 2 181 349
- US-A1- 2005 186 171

## Description

La présente invention concerne une composition cosmétique ou dermatologique qui neutralise les radicaux libres, stimule la microcirculation et la production d'énergie pour dynamiser les cellules et accélérer leur renouvellement, protéger le collagène et lutter contre les taches de pigmentation. Tout particulièrement l'invention concerne une composition cosmétique ou dermatologique caractérisée en ce qu'elle comprend :
(i) au moins des huiles végétales riches en oméga-3 et/ou en oméga-6 choisies dans le groupe comprenant les huiles d'échium, de cameline, d'onagre et de bourrache, lesquelles huiles végétales sont présentes dans une proportion permettant d'obtenir un rapport oméga-3 sur oméga-6 dans ladite composition compris entre 1 et 5,
(ii) au moins un minéral, de préférence sous forme de sel, choisi dans le groupe comprenant le sodium, le calcium et le magnésium,
(iii) au moins un oligoélément choisi dans le groupe comprenant le zinc, le cuivre et le fer,
(iv) au moins de la vitamine C ou un dérivé de celle-ci, et
(v) au moins une huile essentielle, laquelle présente des propriétés anti-radicalaire, ladite huile essentielle est une huile essentielle d'hélichryse (hélichrysum italicum)

Les conditions de la vie moderne font subir à l'organisme diverses agressions permanentes (pollution, métaux lourds, températures extrêmes, U.V., ...).

La peau qui constitue une barrière physique, thermique, mais aussi immunologique entre le corps et les éléments extérieurs, doit ainsi faire face à ces diverses agressions. Parmi celles-ci, une agression majeure résulte de l'action des radicaux libres dont la réactivité chimique entraîne l'altération des différents composants cellulaires de la peau.

Toutefois, le vieillissement de la peau altère son efficacité. Celui-ci ralentit en effet le renouvellement cellulaire de la peau et de sa matrice extracellulaire, notamment le renouvellement du collagène de type I. Ce moindre renouvellement augmente la sensibilité de la peau aux agressions extérieures et sa détérioration avec le temps.

Si différentes compositions ont été mises au point dans l'art antérieur, aucune n'apporte une réponse globale
pour combattre l'ensemble des signes de l'âge et présentant simultanément une action de :
   - stimulation de la prolifération des kératinocytes pour réparer les tissus, laquelle prolifération est accélérée par la stimulation de la production d'énergie et de la microcirculation cutanée ;
   - stimulation de la production de collagène et action anti-collagénase pour une peau plus ferme ; et
   - inhibition de la formation des taches pigmentaires.

Aussi, il existe encore aujourd'hui un besoin important pour l'identification de nouvelles compositions présentant une action globale sur la peau.

Les graisses sont très caloriques et parfois dangereuses, certaines d'entre elles sont tout à fait bénéfiques pour la santé et aujourd'hui nous savons qu'elles constituent des substances essentielles au bon fonctionnement de l'organisme. Les graisses servent ainsi de réserve d'énergie, elles participent à l'architecture des structures vivantes, elles font partie des messagers chimiques : la vie est impossible sans elles. En outre, elle joue un rôle majeur au niveau de la peau puisque lorsque les acides gras des cellules sont endommagés par les radicaux libres, les membranes se flétrissent avec pour premier symptôme clinique une peau sèche et terne.

Le rôle énergétique des graisses est majeur puisqu'elles apportent à poids égal plus de calories que les sucres ou les protéines. Elles constituent ainsi un matériau de base qui permet de stocker l'énergie dans un minimum d'espace.

Les graisses jouent aussi un rôle majeur pour le maintien de l'intégrité du corps : 15 % du poids du corps est constitué de graisses qui ont pour fonction de cimenter les organes et de les maintenir en place.

Elles sont aussi indispensables au développement du cerveau et à sa maturation. Elles permettent de conserver la chaleur corporelle et d'éviter les écarts brutaux de température. Elles apportent une grande partie des acides gras et vitamines liposolubles dont le corps a un besoin absolu pour se construire et rester en bonne santé.

Les graisses assurent l'absorption des vitamines A, E, D, K dites liposolubles. Elles sont la source principale des acides gras essentiels (AGE) que l'organisme ne sait pas synthétiser.

C'est dans les années 1930 qu'est fait le rapport entre les problèmes de peau du type de certains eczémas et un déficit en acides gras dits essentiels qui seront appelés dans un premier temps vitamine F. Cette appellation abandonnée dans certains pays a eu le mérite de souligner l'importance de ce corps gras au niveau de la peau.

Les Acides Gras Essentiels interviennent également sur l'équilibre du système nerveux, la régulation hormonale et métabolique, la circulation du sang, la sexualité, et sur la qualité de la peau et des phanères (cheveux et ongles).

Il existe schématiquement, comme pour les sucres et le cholestérol, de « mauvais » acides gras dits « saturés » qui sont responsables de nombreux problèmes de santé et de « bons » acides gras dits « insaturés » qui ont au contraire une action protectrice.

Plus spécifiquement, les graisses sont réparties en quatre groupes :
- les acides gras saturés ;
- les acides mono-insaturés (encore appelés oméga-9 car leur unique double liaison est située sur le 9^{e} carbone de leur molécule) ;
- les acides poly-insaturés oméga-6 (indiquant la place d'une double liaison chimique sur le 6^{e} carbone de l'acide gras qui en contient entre 4 et 26) ; et
- les acides poly-insaturés oméga-3 (indiquant la place d'une double liaison chimique sur le 3^{e} carbone de l'acide gras).

Parmi ces différents acides gras, deux sont dits « essentiels » :
- l'acide linoléique (oméga-6) ; et
- l'acide alpha-linolenique (oméga-3).

Les autres acides gras dérivent en effet de ces deux acides gras essentiels qui doivent être présents en quantité suffisante et présenter un rapport équilibré. Ainsi, si l'un de ces deux acides gras est présent en quantité trop importante, sa transformation se fera au détriment de l'autre. À titre d'exemple, une consommation excessive d'huile de tournesol favorise la voie des oméga-6 au détriment de la voie des oméga-3, ce qui entraîne une surproduction de substances pro-agrégantes et augmente le risque de thrombose vasculaire. Un apport équilibré en oméga-3 et en oméga-6 est donc essentiel pour maintenir un équilibre corporel. Selon le Conseil Supérieur de l'Hygiène Ce rapport oméga-3/oméga-6 est de 1 à 4 - 6, selon l'OMS (Organisation Mondiale de la Santé) de 1 à 5 et plus, selon la NIH (National Institute of Health) de 1 à 3 et plus. À titre d'exemple, le lait maternel présente un rapport de 1 à 5.

Il est admis par la communauté scientifique que les acides gras essentiels et plus particulièrement les acides gras poly-insaturés (A.G.P.I.) jouent un rôle fondamental dans la construction et le fonctionnement des cellules. Ils constituent une partie de la membrane, assurent son étanchéité et les échanges avec les cellules voisines. Ils stimulent les défenses de l'organisme ; ils améliorent l'apprentissage et la mémoire, participent à la qualité de la vision ; ils nourrissent la peau, lui donnant un meilleur aspect.

Les huiles végétales riches en acides gras poly-insaturés sont en parfaite affinité avec la peau dont elles favorisent les processus vitaux : elles stimulent notamment la formation du film dermo-protecteur qui rend la peau lisse et souple.

Plante longtemps oubliée, l'échium revient au premier plan des plantes médicinales. Sa teneur élevée en Acides Gras Essentiels (AGE), particulièrement en acide alinolénique justifie ses propriétés médicinales et cosmétiques. De la famille des borraginacées, l'échium est une plante à tige dressée, très florifère, ayant la particularité d'être entièrement hérissée de poils raides et piquants, les fleurs sont disposées en grappes et ont une corolle de 1.5 à 2 cm, la floraison ayant lieu de mai à juillet. Cette plante qui pousse de manière sauvage dans les lieux arides peut mesurer de 1 à 3 mètres de hauteur. Une particularité de l'échium est le changement de couleur de ses fleurs en fonction du taux d'acidité du suc cellulaire. La fleur jeune a un suc acide et rouge, la fleur vieillissante a un suc alcalin et bleu. L'huile d'échium se singularise des autres huiles végétales par son fort pourcentage d'acide stéaridonique que l'on trouve principalement dans des huiles de poissons des mers froides.

La cameline (*Camélina sativa* ou sésame d'Allemagne) est une herbe originaire des steppes de l'Asie. Cultivée en Europe depuis la préhistoire, sa culture s'est raréfiée. Plante annuelle de la famille des crucifères, de croissance rapide, elle pousse jusqu'à 1 200 m d'altitude. Sa tige droite rigide atteint 1 m de haut. Ses feuilles plus ou moins velues sont terminées par deux oreillettes pointues. Des petites fleurs jaunes à 4 pétales en croix s'épanouissent de mai à juillet. Leurs grappes porteront en fin d'été des fruits en forme de petite poire, remplis de minuscules graines rougeâtres.

Autre plante riche en AGE, l'onagre, de la famille des onagracées, est une plante à tige dressée, anguleuse, qui porte de grandes fleurs jaunes. Sur la tige se trouvent de nombreux fruits. L'onagre bisannuelle pousse généralement au soleil ou à mi-ombre sur des sols siliceux. Ses feuilles sont simples, longues et duveteuses, avec des nervures. La fleur contient 8 étamines, 4 pétales de 4 à 5 cm plus court que les 4 sépales. Cette fleur s'ouvre en fin de journée, d'où son nom commun de primevère du soir. La floraison a lieu de juin à septembre, après laquelle vient le fruit qui mesure de 2 à 3 cm.

Autre plante riche en AGE, la bourrache (famille des borraginacées) qui est une plante annuelle pouvant atteindre 60 cm et qui est couverte de poils blanchâtres raides et piquants. Ses feuilles alternes sont vert mat, charnues, ondulées avec des feuilles supérieures sessiles embrassant la tige par leur base. Les fleurs sont portées par un long pédoncule velu en forme d'étoile à cinq pétales aigus d'un bleu intense à maturité, rosée lorsqu'elles sont très jeunes. Les fruits sont eux composés de quatre akènes bruns ou noirâtres, sillonnés.

Les éléments organiques ou minéraux (généralement sous forme de sels), principalement le calcium, le sodium et le magnésium, et les oligo-éléments (fer, zinc, cuivre, ...) possèdent également un rôle physiologique important, avec principalement une action sur l'activité enzymatique cellulaire où ils interviennent comme co-facteur en groupements prosthétiques d'enzymes.

Au niveau de la surface cutanée, les sels minéraux sont retrouvés dans les kératinocytes, cellules de l'épiderme, mais aussi dans les fibroblastes qui appartiennent aux cellules du derme. Les sels minéraux rentrent dans la composition du NMF (Natural Moisturising Factor).

Plus spécifiquement, le magnésium est un cation intracellulaire important et un activateur de nombreux systèmes enzymatiques. C'est un minéral essentiel pour le maintien de la bonne santé de l'organisme. Le magnésium intervient ainsi dans le système de transfert des phosphates et dans le système de production de l'énergie (synthèse de l'ATP). Avec l'âge, il apparaît une carence cutanée en magnésium, celui-ci participant à de très nombreuses réactions métaboliques, une supplémentation en magnésium est nécessaire afin de maintenir la peau dans de bonnes conditions physiologiques.

Les oligoéléments sont représentés au niveau du film hydro-lipido-protéinique, d'où leur rôle dans le maintien de l'hydratation cutanée.

Le cuivre est un oligoélément important puisqu'il intervient comme co-facteur dans de nombreuses réactions d'oxydation. Le cuivre intervient ainsi dans le métabolisme oxydatif et dans la respiration cellulaire où il permet la synthèse d'ATP par son rôle de cofacteur dans diverses réactions enzymatiques. Le cuivre présente également une action sur le dynamisme cellulaire, qui est liée à ses fonctions dans la synthèse protéique. Au niveau cutané, le cuivre intervient dans la synthèse de la kératine, protéine majeure de soutien de l'épiderme, des cheveux et des ongles, où il agit comme catalyseur de la formation des ponts di-sulfures. Le cuivre stimule également la production de collagène, protéine de structure du derme, et une carence en cuivre induit une diminution de la formation des liaisons intra-moléculaires, avec pour conséquence une diminution de la synthèse du collagène. Le cuivre intervient enfin dans les mécanismes de défenses cellulaires et plus particulièrement au niveau des enzymes anti-radicalaires comme la Superoxyde Dismutase (SOD).

Le zinc est également un oligoélément important puisqu'il est notamment nécessaire à la bonne croissance cellulaire avec une action sur les systèmes endocriniens, mais également comme bio-catalyseur des réactions enzymatiques, notamment au niveau dermique. La peau renferme une part importante du zinc corporel (environ 20 %) et une carence en zinc est associée à une desquamation importante. Cofacteur d'enzymes Zinc-dépendantes, le zinc stimule la reconstruction de la matrice collagénique et favorise les phénomènes de cicatrisations et la synthèse du collagène dermique. Le zinc permet aussi de lutter contre les phénomènes de vieillissement par la stabilisation des protéines et la stimulation de l'expression du matériel génétique. Le zinc intervient ainsi au niveau de la synthèse de la kératine en contribuant à la formation de ponts intra-moléculaires En favorisant la synthèse et la stabilité de cette protéine de structure, le zinc assure un bon maintien de l'épiderme et de la fibre capillaire.

D'importantes recherches menées par la demanderesse ont mis en évidence qu'une composition comprenant (i) des huiles végétales (échium, cameline, onagre et bourrache), avec un rapport oméga-3 sur oméga-6 compris entre 1 et 5, (ii) des oligoéléments (magnésium, cuivre et zinc), (iii) un dérivé de vitamine C (acétate de tocophéryl) et (iv) une huile essentielle (Hélichryse) présentent une action synergique et globale sur la peau.

En conséquence, un premier objet de l'invention consiste en une composition cosmétique ou dermatologique caractérisée en ce qu'elle comprend :
(i) au moins des huiles végétales riches en oméga-3 et/ou en oméga-6 choisies dans le groupe comprenant les huiles d'échium, de cameline, d'onagre et de bourrache, lesquelles huiles végétales sont présentes dans une proportion permettant d'obtenir un rapport oméga-3 sur oméga-6 dans ladite composition compris entre 1 et 5,
(ii) au moins un minéral, de préférence sous forme de sel, choisi dans le groupe comprenant le sodium, le calcium et la magnésium, de préférence le magnésium,
(iii) au moins un oligoélément choisi dans le groupe comprenant le zinc, le cuivre et le fer, de préférence dans le groupe comprenant le zinc et le cuivre,
(iv) au moins de la vitamine C ou un dérivé de celle-ci, et
(v) au moins une huile essentielle, laquelle présente des propriétés anti-radicalaires, **ladite huile essentielle étant** une huile essentielle d'hélichryse.

La composition selon l'invention permet de stimuler la production d'énergie et la prolifération des kératinocytes, de neutraliser les radicaux libres, de stimuler la micro circulation, donc d'effectuer un traitement en profondeur.

Par « riche » en oméga-3 et/ou en oméga-6, on entend au sens de la présente invention une huile présentant une concentration supérieure à 10% en oméga-3 et/ou en oméga-6.

L'homme du métier pourra identifier simplement au regard de ses seules connaissances générales et à l'aide d'expérience de routine des huiles végétales riches en oméga-3 et/ou en oméga-6. De même, l'homme du métier sera à même d'ajuster simplement la proportion de chacune de ces huiles d'obtenir un rapport oméga-3 sur oméga-6 dans ladite composition compris entre 1 et 5. Ainsi, et à titre d'exemple :
a) L'huile d'échium et plus spécifiquement l'huile de graines d'échium possède une concentration moyenne de 30 % d'acide alpha-linolénique (oméga-3).
b) L'huile de caméline et plus spécifiquement l'huile de graines de caméline possède une concentration moyenne de 35 % en acide alpha-linolénique (oméga-3).
c) L'huile d'onagre et plus spécifiquement l'huile de graines d'onagre possède une concentration moyenne de 10 % d'acide gamma-linolénique (oméga-6) et 70 % d'acide linoléique.
d) L'huile de bourrache et plus spécifiquement l'huile de graines de bourrache possède une concentration moyenne de 19 % d'acide gamma-linolénique (oméga-6) et 35 % d'acide linoléique.

Selon un premier mode de réalisation préféré, la composition selon l'invention comprend des huiles d'échium, de caméline, d'onagre et de bourrache et de manière particulièrement préférée des huiles de graines d'échium, de graines de caméline, de graines de l'onagre et de graines de bourrache.

Les huiles végétales d'échium, de cameline riches en oméga-3, d'onagre et de bourrache riches en oméga-6 ont été choisies en raison de leur teneur relativement constante en principe actif ce qui permet d'ajuster facilement les pourcentages pour respecter le rapport oméga-3 / oméga-6 compris entre 1 à 5. En outre, l'apport d'antioxydants et d'acides gras procurés par les huiles de bourrache, d'onagre, d'échium et de cameline constitue une mesure efficace pour récupérer ou obtenir rapidement une belle peau. Dans la composition selon l'invention, l'huile d'échium, de par sa composition en AGE, stimule activement à la régénération des membranes cellulaires et lutte efficacement contre le dessèchement de la peau.

L'homme du métier pourra déterminer sans difficulté les quantités des différentes nécessaires pour obtenir l'effet souhaité.

Avantageusement, la composition selon l'invention comprend une teneur en :
a) huile d'échium comprise entre 0,1 % et 10 % du poids total de la composition, de préférence entre **0,1** et 5 %, et/ou
b) huile de cameline comprise entre 0,1 % et 10 % du poids total de la composition, de préférence entre **0,1** et 5 % et/ou
c) huile de bourrache comprise entre 0,1 % et 10 % du poids total de la composition, de préférence entre **0.1** et 5 %, et/ou
d) huile d'onagre comprise entre 0,1 % et 10 % du poids total de la composition, de préférence entre **0,1** et 5 %.

Les huiles essentielles correspondent à des ensembles complexes de produits odorants retirés de divers organes de plantes aromatiques (racines, fleurs, fruits, feuilles) par distillation ou expression, de préférence par distillation. L'homme du métier, au regard de ses connaissances générales pourra identifier sans difficulté les huiles essentielles présentant des propriétés anti-radicalaires.

Selon un deuxième mode de réalisation préféré, la composition selon l'invention comprend une huile essentielle d'hélichryse (hélichrysum italicum), de préférence une huile d'hélichryse obtenue par distillation à la vapeur d'eau des sommités fleuries.

Avantageusement, la composition comprend une teneur en huile essentielle d'hélichryse comprise entre 0,05 % et 5 % du poids total de la composition, de préférence entre **0,05** et **2,5** %.

Selon un troisième mode de réalisation préféré, la composition selon l'invention comprend du zinc, du cuivre et du magnésium.

En effet, il apparaît que ces trois composants stimulent les cellules en augmentant le bilan énergétique (ATP) et le métabolisme basal.

À titre d'exemple, le zinc, le cuivre et le magnésium sont ajoutés à la composition selon l'invention sous la forme du cocktail de minéraux à action énergisante « Sepitonic M3 »disponible chez SEPPIC.

Avantageusement, la composition selon l'invention comprend une teneur :
a) du magnésium comprise entre 0,001 % et 5 % du poids total de la composition, de préférence entre **0,001** et 1 %, et/ou
b) du cuivre comprise entre 0,001 % et 5 % du poids total de la composition, de préférence entre 0.001 et 1 %, et/ou
c) du zinc comprise entre 0,001 % et 5 % du poids total de la composition, de préférence entre **0,001** et 1 %.

La vitamine C (acide ascorbique) et ses dérivés sont bien connus de l'homme du métier.

Avantageusement, la composition selon l'invention présente une teneur en vitamine C ou en l'un de ses dérivés comprise entre **0,5** % et 10 % du poids total de la composition, de préférence entre **0,5** % et 5 %.

Selon un quatrième mode de réalisation préféré, la composition selon l'invention comprend, à titre de dérivé de la vitamine C, de l'acide ascorbique 2-glucoside (Ascorbic Acid 2-Glucoside ou O-α-D-Glucopyranosyl-(1→2)-L-ascorbic acid).

La demanderesse a en effet mis en évidence que l'adjonction d'un tel dérivé de vitamine C permettait d'une part de réduire la mélanine déjà produite et, d'autre part, d'inhiber la production de nouvelle mélanine.

Un dérivé de vitamine C stabilisée avec du glucose se combine avec une enzyme présente dans la peau, l'alfaglucosidase, pour permettre une libération retardée de l'actif. Cette libération lente va réduire les taches de pigmentation dues à l'âge en inhibant la production de la mélanine.

Un tel dérivé de vitamine C est notamment disponible chez HAYASHIBARA.

Les compositions selon l'invention peuvent se présenter sous toutes les formes galéniques classiquement utilisées, et bien connues de l'homme de l'art, dans les domaines cosmétiques ou dermatologiques pour être appliquées sur la peau, comme par exemple des émulsions Huile dans Eau (H/E) ou Eau dans Huile (E/H), des lotions, sprays, gels, laits, crèmes, etc., sans aucune restriction galénique particulière autre que celles de la compatibilité de la composition avec l'excipient.

En outre, les compositions de l'invention peuvent aussi contenir tous les constituants classiquement utilisés dans ces présentations comme des agents de formulation, des agents conservateurs, des agents solubilisants, des agents gélifiants, des tensio-actifs, des corps gras, du propylène glycol, du sorbitol, du glycérol, des bases de parfums et de l'eau.

La composition selon l'invention peut comprendre en outre au moins un autre actif choisi dans le groupe comprenant les agents affinant, restructurant, anti-inflammatoire, hydratant et antiseptique de la peau.

Avantageusement, la composition selon l'invention comprend alors une teneur en ledit actif comprise entre 1 est 20% du poids total de la composition, de préférence entre 1 et 10%.

Un deuxième objet de l'invention correspond à une méthode de soin cosmétique comprenant une étape d'application sur la peau d'un sujet d'une quantité efficace d'une composition telle que définie précédemment.

La méthode de soin selon l'invention permet de stimuler la production d'énergie, la prolifération cellulaire, la neutralisation des radicaux libres et la microcirculation, de stimuler et protéger la production de collagène, de réduire et inhiber la production de mélanine.

Un troisième objet de l'invention correspond à l'utilisation cosmétique d'une composition telle que décrite précédemment pour opérer un traitement en profondeur qui stimule la production d'énergie, dynamise et accélère le renouvellement cellulaire et inhibe la production de mélanine.

La composition selon l'invention protège la peau contre l'attaque des radicaux libres et freine le vieillissement.

D'autres caractéristiques et avantages de la présente invention apparaîtront à la lecture des exemples non limitatifs qui suivent.

### Exemple 1 : Crème complète - Soin visage multiaction

| | |
|---|---|
| Stéarate de sucrose | 0,5 à 6 % |
| Distéarate de sucrose | 0,5 à 6 % |
| Triglycéride caprylique /caprique % | 3 à 15 |
| Triglycéride caprylique/caprique/succinique % | 3 à 15 |
| Huile d'échium | 0,1 à 5 % |
| Huile de cameline | 0,1 à 5 % |
| Huile de bourrache | 0,1 à 5 % |
| Huile d'onagre | 0,1 à 5 % |
| Magnésium | 0,001 à 1 % |
| Cuivre | 0,001 à 1 % |
| Zinc | 0,001 à 1 % |
| HE d'Hélichryse | 0,05 à 2,5 % |
| Ascorbic Acid 2- Glucoside | 0,5 à 5 % |
| Acétate de tocophéryle | 0.05 à 1 % |
| Urée | 0,5 à 2 % |
| Glycérine | 0,5 à 3 % |
| Hyaluronate de sodium | 0,5 à 3 % |
| Sorbate de potassium | 0,2 à 0,5 % |
| Gomme xanthane | 0,1 à **0,5** % |
| Parfum | qs |
| Conservateurs | qs |
| Eau | qsp 100 % |

### Exemple 2 : Activité anbi-radicalaire

### 1) Objectif de l'étude

Le radical hydroxyle est une molécule très réactive dont la production est activée par les différents stress subits par la peau (pollution, UV, variation de température, etc.). Ce radical est responsable de nombreux dégâts dans la cellule et notamment de la dégradation des membranes par le phénomène de péroxydation lipidique.

L'objectif de la présente étude a été d'apprécier le pouvoir anti-radicalaire de l'huile essentielle d'hélichryse (Helichrysum Italicum) par la méthode in vitro des diènes conjugués. Cette méthode permet de tester l'action protectrice de ladite huile essentielle sur des liposomes (analogues de membranes) soumis à une attaque de radicaux hydroxyles (OH-)

### 2) Principe de l'étude

L'action des radicaux libres sur les acides gras polyinsaturés des membranes correspond à une peroxydation conduisant à la formation de diènes conjugués absorbant à 233 nm.

Cette peroxydation est donc caractérisée par un pic d'absorption à 233 nm.

L'activité des antioxydants peut donc être dosée par leur capacité à empêcher la péroxydation d'acides gras polyinsaturés contenus dans des liposomes (modèle in vitro se rapprochant le plus de la réalité in vivo).

### 3) Matériels et méthodes

### Réactifs :

- Tampon phosphate **0,05** M pH 6,8 (Na2HPO4 : **3,55** g ; KH2PO4 : **3,4** g ; H2O qsp 1000 ml)
- Solution de potassium thiocynate **0,03** M (KSCN : 0,3 g ; tampon phosphate qsp 100 ml)
- Suspension de liposomes (Natipides : 3 g ; Glydant (conservateur) : 0,3 g; Eau distillée qsp 100 ml)
- Solution de FeCl₂ à **2,3** x 10⁻³ M
- Solution de H₂O₂ à **110** volumes diluée au 10^{ème}
- Solution de CaCl₂ à **0,5** M
- Heptane

### Protocole :

L'activité d'une solution comprenant **0,1** % d'huile essentielle d'hélichryse a été dosée simultanément à une solution témoin. Après homogénéisation, les solutions ont été laissées dans une étuve à 45 ° C durant une nuit. 5 ml de chaque solution ont alors été prélevés. Les lipides ont été précipités par addition de 5 ml de CaCl₂.

Ce mélange a été agité pendant 10 minutes à l'aide d'un barreau aimanté, puis les lipides ont été extraits en ajoutant 10 ml d'heptane et en agitant pendant 15 minutes.

Le spectre UV (200 à 350nm) de chaque solution a été ensuite enregistré contre de l'heptane, dans des cuves en quartz. Un pic à 233 nm traduit la présence de diènes conjugués et permet de quantifier ces derniers par rapport au témoin.

### Résultats :

Au terme de l'essai, il apparaît que le produit Huile essentielle d'hélichryse (Helichrysum Italicum) inhibe la peroxydation lipidique de 82 % ± 2 dans les conditions expérimentales.

### Exemple 3 : Stimulation de la synthèse de l'ATP

L'énergie cellulaire, nécessaire à toute activité, se présente sous forme d'ATP. La production d'ATP est en partie assurée par la dégradation des glucides simples comme le glucose, en pyruvate (glycolyse).

### 1) Principe du test

Après 6 heures d'incubation, le contenu intracellulaire en ATP a été mesuré par bioluminescence à l'aide du kit « ATP Monitoring kit » (LABSYSTEMS) selon les instructions du fabricant. Les résultats ont été exprimés en mM d'ATP et en pourcentage du groupe témoin.

Effet du Sepitonic M3 et du cytochrome C sur le contenu en ATP de kératinocytes après 6 heures d'incubation :
Cytochrome C 0,01%: ES 110%
Sepitonic M3 0,001%: ES 128%

Le pyruvate produit initialement se transforme en acetyl CoA , ce dernier , via le cycle de l'acide citrique réalisé dans les mitochondries,produit une grande quantité de NADH ,H + .Lors de la respiration cellulaire, le NADH, H+ sert de source donneuse d'électrons . Tout au long de la chaîne de transport d'électrons (et de phosphorylation oxydative) l'énergie libérée permet la synthèse d'ATP .

En conclusion, le Sepitonic M3 dynamise les cellules en stimulant le métabolisme énergétique cellulaire avec une efficacité supérieure à la référence à une concentration 10 fois moindre. La production d'ATP dans les kératinocytes est alors augmentée de 32 % par rapport au témoin. Cet ATP « complémentaire » constitue une réserve d'énergie supplémentaire pour améliorer le bon fonctionnement des cellules.

### Exemple 4 Exploration de l'activité proliférative d'un complexe huileux oméga 3 et oméga 6 sur les structures épidermiques après multiples applications sur explants de peau humaine maintenus en survie

### 1) But de l'étude

Cette étude a pour but d'explorer l'activité proliférative d'un complexe huileux sur explants de peau humaine maintenus en survie.

L'activité a été évaluée par une expertise histologique de la morphologie générale de la peau après coloration au trichrome de Masson et par immunomarquage des cellules en mitoses (KI 67) (index mitotique).

### 2) Mode opératoire

### Préparation des explants

Préparation de 15 explants de peau humaine et mise en survie en milieu spécifique. Les explants ont été répartis en 2 lots de six explants et un lot témoin T0 de trois explants, comme suit :

| | | |
|---|---|---|
| T0 | Témoin Plastie | 3 explants |
| T | Témoin non traité | 6 explants |
| P1 | Explants traités par le complexe à tester | 6 explants |

### Traitement

Le traitement a été réalisé à J0, 2 heures après la préparation des explants, puis à J1, J2, J4, J6 et J8.

Les produits ont été appliqués sur les explants comme suit :
T les explants n'ont reçu aucun traitement,
P1 les explants ont reçu chacun 25 ml du complexe huileux déposé sur un disque papier et appliqué sur chaque explant.

Le traitement a été réalisé par application topique du produit à tester. Le produit est ensuite réparti sur toute la surface de l'explant, à l'aide d'une spatule. La moitié du milieu de culture a été renouvelé tous les deux jours et les explants ont été remis en survie à 37 °C en atmosphère humide enrichie de 5% de CO2

### Prélèvement pour l'histologie

À J3 et J9, 3 explants de chaque lot ont été prélevés. Les prélèvements ont été coupés en deux, une moitié a été fixée dans du Bouin ordinaire et l'autre moitié congelée à -80°C.

### Étude morphologique

Après 24 heures de fixation dans le Bouin ordinaire, les prélèvements ont été déshydratés, imprégnés en paraffine, mis en blocs et des coupes de 5 µm ont été réalisées. L'étude morphologique des structures épidermiques et dermiques a été réalisée sur les coupes colorées au trichrome de Masson, variante de Goldner.

### Immunomarquage du KI 67

Sur les prélèvements congelés, des coupes à 7 mm ont été réalisées. L'immunomarquage des cellules en mitoses a été réalisé sur coupes congelées avec l'anti-corps polyclonal anti-KI 67 (Novo Castra) révélé en DAB (Di Amino Benzédine).

Les cellules positives ont été comptées sur toute la longueur épidermique et les moyennes ramenées au nombre de cellules marquées par centimètre.

### Résultats

### Morphologie générale

Témoin T0 : Le stratum corneum est épais, compact, assez nettement kératinisé en surface et à sa base. La structure épidermique présente 4 à 5 assises cellulaires présentant une bonne morphologie. Le relief de la jonction dermo-épidermique est net. Le derme papillaire montre un collagène plus ou moins fin, assez dense et est bien cellularisé.

Témoin à J3 : Le stratum corneum est épais, très légèrement feuilleté, modérément kératinisé en surface et légèrement à sa base. La structure épidermique présente 5 à 6 assises cellulaires présentant une bonne morphologie. Le relief de la jonction dermo-épidermique est net. Le derme papillaire montre un collagène plus ou moins fin, plus ou moins dense et est bien cellularisé.

Complexe d'acide gras oméga 3 / oméga 6 à J3 : Le stratum corneum est épais, assez compact, modérément kératinisé en surface et légèrement à sa base. La structure épidermique présente 3 à 4 assises cellulaires. Le relief de la jonction dermo-épidermique est net. Le derme papillaire montre un collagène plus ou moins fin, peu dense et est bien cellularisé.

Témoin à J9 : Le stratum corneum est épais, assez compact, légèrement kératinisé en surface et plus nettement à sa base. La structure épidermique présente 4 à 5 assises cellulaires présentant une bonne morphologie avec une légère spongiose. Le relief de la jonction dermo-épidermique est net. Le derme papillaire montre un collagène plus ou moins fin, assez dense et est bien cellularisé.

Complexe d'acide gras oméga 3 / oméga 6 à J9 : Le stratum corneum est épais, assez feuilleté, nettement kératinisé en surface avec une parakératose modérée. La structure épidermique présente 4 à 5 assises cellulaires. Le relief de la jonction dermo-épidermique est net. Le derme papillaire montre un collagène plus ou moins fin, plus ou moins dense et est bien cellularisé.

### Cellules en mitose :

Les résultats des comptages des cellules en mitose sont présentés dans le tableau suivant :

| | Témoin | Complexe |
|---|---|---|
| J0 | 180 | 180 |
| J3 | 25 | 80 |
| J9 | 25 | 20 |

### Discussion

### Morphologie générale :

À J3 et J9, la structure épidermique est normale sur les explants non traités.

Sur ceux traités avec le complexe d'acides gras oméga 3 / oméga 6, comparativement aux explants non traités, le stratum cornéum est nettement feuilleté surtout à J9. L'épaisseur épidermique est plus mince à J3 et comparable à J9.

### Index mitotique :

À J3, l'indexe mitotique montre une très nette augmentation du nombre de cellules en mitose sur les explants traités avec le complexe d'acides gras oméga-3 / oméga-6 par rapport aux explants témoins.

### Conclusion

Dans ces conditions opératoires, le complexe d'acides gras oméga-3 /oméga-6 montre à J3 une nette activité stimulante sur les kératinocytes.

La peau Témoin mise en survie a une baisse normale des cellules en mitose: La stimulation se mesure par le rapport entre 80 et 25.

Sans actif, cette baisse est, de 86 %. Avec le complexe huileux, elle n'est plus que de 56 %. On peut en déduire qu'il y a une stimulation de 30 % de la division cellulaire au bout de 3 jours.

### Exemple 5 : Action de l'Ascorbic Acid 2-Glucoside (AA-2G) sur la réduction de la mélanine.

### 1) Méthode

a) Culture de mélanocytes pendant 48 heures
b) Ajout de l'AA-2G à 2 mM pendant 48 heures
c) Lyse des cellules pour lecture de la quantité de la mélanine contenue.

### 2) Résultats :

Les résultats ont montré une diminution de 60 % ± 2 de la mélanine intra-cellulaire en présence d'AA-2G.

### Example 6 : Inhibition de la mélanogénèse par l'AA-2G

### 1) Méthode

a) Mise en culture pendant 12 heures des mélanocytes avec l'AA-2G à **2,5** mM + témoin sans AA-2G.
b) Stimulation de la mélanogénèse par la théophylline (0.5 mM)
c) Mise en évidence par colorimétrie de la mélanine (photos)

### 2) Résultats :

Les photos des mélanocytes traités ont montré une quantité de mélanine développée moins importante que sur les mélanocytes non traités.

### Exemple 7 : Evaluation de l' activité anti-collagénane

### 1) But de l'étude

Le but de cette étude est de montrer l'activité anti-collagénase d'une huile essentielle et d'une formulation contenant une huile essentielle.

### 2) Mode opératoire

Le test d'activité anti-collagénase a été réalisée sur ds-es coupes congelées. Les produits à tester ont été mélangés (volume à volume) avec une solution de collagénase à 60U/ml. L'incubation a été réalisée en chambre humide pendant 2 H à 37 °C. Le collagène subsistant a été coloré au picro-sirius et quantifié en analyse d'images.

Lot :
1. Témoin tampon TRIS
2. Collagénase à 300/ml
3. Référence R : Phénanthroline
4. Référence R : Phénanthroline + collagénase à 60U/ml
5. produit P2 : Crème très précieuse immortelle ref 05.34.A
6. Produit P2 : Crème très précieuse immortelle ref 05.34.A + collagénase à 60U/ml

### 3) Examen histologique

### Témoin tampon TRIS

Le collagène observé est bien structuré.

### Collagénase :

Le collagène observé est fortement déstructuré.

### Phénanthroline à 1,25 % :

Le collagène observé est bien structuré.

### Phénanthroline à 1,25 % + collagénase :

Le collagène observé est bien structuré.

Crème très précieuse immortelle (**0,12** % d'hélichryse) :

Le collagène observé est bien structuré.

### Crème très précieuse immortelle + Collagénase :

Le collagène observé est bien structuré.

### 4) Mesures de la coloration en analyse d'image

Pour chaque prélèvement, 4 champs microscopiques sont analysés en fonction de la taille du prélèvement. Chaque champ microscopique est numérisé et analysé suivant la méthode décrite ci-dessous par le logiciel LEICA QWIN.

### 1. Image numérisée

1. Image numérisée
   Coloration du réseau de collagène
2. Détection du collagène par seuillage
   Masque binaire 0
3. Détermination de la zone d'intérêt
   Exclusion de l'épiderme et des annexes
   Inversion du masque pour ne conserver que la zone correspondant au derme
   Masque binaire 2
4. Sélection du collagène dans la zone d'intérêt
   Masque binaire 3 = 2 + 0
5. Mesure de surfaces
   Mesure du masque binaire 3
   Mesure du masque binaire 2
6. Résultats
   Excel
   Exportation des résultats vers calcul du pourcentage de surface occupé par le collagène dans le derme

Les résultats obtenus en pourcentage de surface occupée par le collagène dans le derme

| | Sans collagénase Moyenne (Ecart type) | Avec collagénase Moyenne (Ecart type) |
|---|---|---|
| Témoin (tampon TRIS) | **88, 3** (2,9) < | **41,6** (**2,1**) |
| Référence positive (phénanthroline 1.25%) | **88,7** (1,5) | **90,4** (**3,8**) |
| P2 Crème très précieuse Immortelle | **86,0** (4,4) | **88,0** (2.9) |

### Discussion

La phénanthroline, testée à **1,25** % en tant que référence positive, inhibe totalement l'effet de la collagènase, validant ainsi l'étude. Les résultats montrent que la crème très précieuse immortelle inhibe totalement l'action de la collagénase dans une proportion proche de la phénanthroline.

### Conclusion

Dans les conditions opératoires décrites ci-dessus, le produit « Crème très précieuse immortelle » présente une forte activité anti-collagènase.

### Excample 8 : Etude de l'effet de la composition sur la synthèse de collagène de type 1

### 1) Introduction :

- Les fibres du derme : Les fibres du derme comprennent les fibres de collagène et les fibres élastiques. Elles représentent quantitativement les protéines de structures les plus importantes du derme, soit respectivement 75 % et 5 % de leur poids sec. Leur proportion relative et leur disposition sont différentes selon les zones superficielles ou profondes du derme.
- Les fibroblastes : les fibroblastes sont responsables de la synthèse et de l'entretien du matériel extracellulaire. Ce sont des cellules d'origine mésenchymateuse, qui synthétisent le collagène, l'élastine, la substance fondamentale et les glycoprotéines de structure.
- Les fibres de collagène : les collagènes forment une très grande famille. Ce sont des molécules de la matrice extracellulaire composées de trois chaînes polypeptidiques portant la répétition de trois acides aminés : - Gly-X-Y, où X et Y sont souvent des prolines ou des hydroxyprolines. Les fibres de collagène du derme sont constituées respectivement de collagène I et de collagène III, autour d'un axe composé du collagène V. Ces collagènes appartiennent au groupe des collagènes fibrillaires. Chez l'adulte, le collagène I est en moyenne six fois plus abondant que le collagène III.
- Collagènes et vieillissement : Le rapport collagène I/collagène III diminue au cours du vieillissement. On assiste à une augmentation exponentielle de pontages chimiques entre les fibres de collagène dus à la réaction de glycation non enzymatique de Maillard. Ce pontage chimique du collagène aboutit à une rigidification des fibres. Sa dégradation et son renouvellement sont ainsi ralentis.
- Modification des fibroblastes : Le fibroblaste est une cellule clef du tissu conjonctif qui intervient dans la formation et la stabilisation des fibres élastiques, mais aussi dans leur dystrophie et leur lyse. Lors du vieillissement, le fibroblaste diminue son activité et cette cellule au repos est appelée fibrocyte. Il devient globuleux avec diminution de son cytoplasme et raréfaction de son réticulum endoplasmique dont les vésicules sont très dispersées. Cette cellule n'est plus en contact avec le collagène.

### 2)_principe de l' étude

La modification favorisant la perte d'élasticité et de fermeté de la peau du fait de la désorganisation et de la raréfaction du collagène a été étudiée de façon à démontrer l'efficacité des compositions de l'invention.

Ainsi, l'activité de la composition sur la sécrétion du collagène I dans le milieu de culture par les fibroblastes a été évaluée.

Le modèle expérimental retenu consiste en une culture de fibroblastes humains normaux jusqu'à confluence. Les cellules sont incubées avec la composition à 0.05 % (concentration révélée non cytotoxique pour les cellules au test MTT). Par la méthode ELISA (Enzyme-Linked Immuno Sorbent Assay), qui présente l'avantage d'une mise en oeuvre facile, sensible et spécifique, nous dosons le collagène de type 1 au niveau du milieu de culture.

### 3) Matériel et méthode

Dans un premier temps des cultures de fibroblastes ont été établies par la méthode des explants à partir de prélèvements de peau caucasienne saine (plastie abdominale). Les fibroblastes sont cultivés jusqu'à confluence, à 37°C dans un milieu MEM (Minimum Essential Medium) contenant 2 mM de L-Glutamine et 10 % de sérum de veau foetal dans une atmosphère saturée en humidité avec 5% de CO2. Puis, après décollement par une solution isotonique tamponnée pH 7.2 de trypsine à **0,1** % et d'EDTA **0,02** %, les cellules sont ensemencées dans des microplaques de 96 puits (Falcon) à 10⁴ cellules par puits dans le milieu susnommé. Après 24 heures, ce milieu est remplacé, pour une incubation de 48 heures par le même milieu sans sérum contenant **0,15** mM d'ascorbate de sodium (milieu d'incubation) avec ou sans le complexe d'actifs à tester.

Un type de fibroblastes a été choisi pour l'étude. Il s'agit de fibroblastes au 4^{ème} passage ou repiquage de culture, représentant des cellules ayant des caractéristiques normales (aspect fusiforme ou étoilé, bonne activité métabolique, bonne capacité d'étalement, etc.).

La quantité de collagène de type I sécrétée dans le milieu après incubation avec ou sans le complexe d'actifs a ensuite été déterminée à l'aide d'un test ELISA. Cette méthode permet de détecter certaines molécules non-radioactives à de très faibles doses. Une méthode directe a été choisie consistant en l'absorption de l'antigène (collagène de type I) à une phase solide (plastique d'une plaque de microtitration réservée à l'ELISA). Pour cela, le milieu d'incubation est collecté et transféré dans les puits d'une plaque de microtitration. Une incubation de 24 heures à +4°C permet l'adhésion du collagène au plastique. La plaque est ensuite rincée au PBS (Phosphate Buffer Saline). Après 24 H d'incubation à + 4°C avec de l'albumine sérique (2% dans PBS) pour éviter les liaisons anti-corps s-plastique non spécifiques, des anti-corps de souris anticollagène I (sigma) (dilution 1/2000) conjugués à une phosphatase alcaline (2 heures à TA) qui en présence du phosphate de paranitrophényl, produira du paranitrophénol absorbant à 405 nm (1 heure à TA). Les densités optiques obtenues sont converties en ng de collagène grâce à une courbe d'étalonnage établie dans les mêmes conditions expérimentales avec du collagène de type I.

### 4) Résultats :

Les résultats ont montrés que la composition utilisée à 0.05% dans le milieu de culture permet aux fibroblastes de synthétiser jusqu'à 6 fois plus de collagène de type I par rapport au témoin (milieu de culture seul).

La composition cosmétique initie et dynamise les programmes de synthèse d'un des composants majoritaires de la matrice extracellulaire du derme, le collagène de type I. La composition agit donc comme un excellent « booster » de la réorganisation des fibres de collagène et permet ainsi d'améliorer la tonicité de la peau.

### Exemple 9 : Evaluation de la crème très précieuse

### 1) Objectif :

Evaluation de l'efficacité anti-rides du produit.

### 2) Critères d'évaluation :

Analyse du relief cutané à partir d'empreintes cutanées.

### 3) Mode d'application :

Le produit est appliqué sur l'ensemble du visage nettoyé chez 44 volontaires tous les soirs pendant huit semaines. Le détail d'une mode d'application est détaillé ci-dessous.

| | |
|---|---|
| J0 - une semaine | - Vérification des critères d'inclusion et de non inclusion |
| | - Signature du consentement |
| | - Remise du produit de référence |
| J0 - une semaine - J0 | Application du produit de référence à domicile deux fois par jour (matin et soir) sur le visage |
| J0 | - Réalisation d'une empreinte cutanée. |
| | - Remise du soin crème très précieuse. |
| J0 - J28 | - Application du produit à domicile une fois par jour (le soir) sur le visage et application du produit de référence une fois par jour (le matin sur le visage. |
| J 28 | - Vérification du respect du protocole. |
| | - Evaluation de la tolérance. |
| | - Recueil des événements indésirables et traitements concomitants. |
| | - Réalisation de l'empreinte cutanée. |
| J 28 - J 56 | - Application du produit à domicile une fois par jour (le soir) sur le visage et application du produit de référence une fois par jour (le matin sur le visage. |
| J 56 | - Vérification du respect du protocole. |
| | - Recueil du produit et de la fiche de suivi. |
| | - Evaluation de la tolérance. |
| | - Recueil des événements indésirables et traitements concomitants. |
| | - Réponse au questionnaire d'acceptabilité cosmétique |
| | - Réalisation de l'empreinte cutanée. |

### 4) Résultats :

Dès quatre semaines, diminution du nombre de rides de 16 % et de la surface totale ridée de 26 % (amélioration moyenne mesurée sur 25 volontaires).

Après huit semaines d'utilisation, l'efficacité est ressentie. Les résultats obtenus sont décrits ci-dessous.

| | |
|---|---|
| La peau est plus ferme | 89 % |
| La peau est plus lisse | 87 % |
| La peau semble régénérée | 89 % |
| Les rides sont atténuées | 85 % |

## Revendications

1. Une composition cosmétique ou dermatologique **caractérisée en ce qu'**elle comprend :
(i) au moins des huiles végétales riches en oméga-3 et/ou en oméga-6 choisies dans le groupe comprenant les huiles d'échium, de cameline, d'onagre et de bourrache, lesquelles huiles végétales sont présentes dans une proportion permettant d'obtenir un rapport oméga-3 sur oméga-6 dans ladite composition compris entre 1 et 5,
(ii) au moins un minéral, de préférence sous forme de sel, choisi dans le groupe comprenant le sodium, le calcium et le magnésium,
(iii) au moins un oligoélément choisi dans le groupe comprenant le zinc, le cuivre et le fer,
(iv) au moins de la vitamine C ou un dérivé de celle-ci, et
(v) au moins une huile essentielle, laquelle présente des propriétés anti-radicalaire, ladite huile essentielle est une huile essentielle d'hélichryse (hélichrysum italicum)

2. La composition selon la revendication 1, dans laquelle ladite composition comprend une teneur en huile essentielle d'hélichryse comprise entre 0,05 % et 5 % du poids total de la composition.

3. La composition selon la revendication 1, dans laquelle ladite composition comprend une teneur en huile essentielle d'hélichryse comprise entre 0,05 et 2,5 % du poids total de la composition.

4. La composition selon l'une quelconque des revendications précédentes dans laquelle le minéral est le magnésium.

5. La composition selon l'une quelconque des revendications précédentes dans laquelle ledit au moins un oligoélément choisi dans le groupe comprenant le zinc et le cuivre

6. La composition selon l'une quelconque des revendications précédentes, dans laquelle ladite composition comprend des huiles d'échium, de caméline, d'onagre et de bourrache.

7. La composition selon l'une quelconque des revendications précédentes, dans laquelle ladite composition comprend une teneur en :
a) huile d'échium comprise entre 0,1 % et 10 % du poids total de la composition, et/ou
b) huile de cameline comprise entre 0,1 % et 10 % du poids total de la composition, et/ou
c) huile de bourrache comprise entre 0,1 % et 10 % du poids total de la composition, et/ou
d) huile d'onagre comprise entre 0,1 % et 10 % du poids total de la composition.

8. La composition selon la revendication 7 dans laquelle ladite composition comprend une teneur en huile d'échium comprise entre 0,1 et 5 % du poids total de la composition.

9. La composition selon la revendication 7 ou 8 dans laquelle ladite la composition comprend une teneur en huile de cameline comprise entre 0,1 et 5 % du poids total de la composition.

10. La composition selon l'une quelconque des revendications 7 à 9 dans laquelle ladite composition comprend une teneur en huile de bourrache comprise entre 0,1 et 5 % du poids total de la composition.

11. La composition selon l'une quelconque des revendications 7 à 10 dans laquelle ladite composition comprend une teneur en huile d'onagre comprise entre 0,1 et 5 % du poids total de la composition.

12. La composition selon l'une quelconque des revendications précédentes, dans laquelle ladite composition comprend une teneur en :
a) magnésium comprise entre 0,001 % et 5 % du poids total de la composition, et/ou
b) cuivre comprise entre 0,001 % et 5 % du poids total de la composition, et/ou
c) zinc comprise entre 0,001 % et 5 % du poids total de la composition.

13. La composition selon la revendication 12, dans laquelle ladite composition comprend une teneur en magnésium comprise entre 0,001 et 1 % du poids total de la composition.

14. La composition selon la revendication 12 ou 13, dans laquelle ladite la composition comprend une teneur en cuivre comprise entre 0,001 et 1 % du poids total de la composition.

15. La composition selon l'une quelconque des revendications précédentes, dans laquelle ladite composition comprend une teneur en zinc comprise entre 0,001 et 1 % du poids total de la composition.

16. La composition selon l'une quelconque des revendications précédentes, dans laquelle ladite composition comprend du zinc, du cuivre et du magnésium.

17. La composition selon l'une quelconque des revendications précédentes, dans laquelle ladite composition comprend une teneur en vitamine C ou l'un de ses dérivés comprise entre 0.5 % et 10 % du poids total de la composition.

18. La composition selon la revendication 17, dans laquelle ladite la composition comprend une teneur en vitamine C ou l'un de ses dérivés comprise entre de entre 0,5 % et 5 % du poids total de la composition.

19. La composition selon l'une quelconque des revendications précédentes, dans laquelle ladite composition comprend un dérivé de la vitamine C, de préférence de l'acide ascorbique 2-glucoside (Ascorbic Acid 2-Glucoside ou *O*-α-D-Glucopyranosyl-(1→2)-L-ascorbic acid).

20. La composition selon l'une quelconque des revendications précédentes, dans laquelle ladite composition comprend en outre au moins un autre actif choisi dans le groupe comprenant les agents affinant, restructurant, anti-inflammatoire, hydratant et antiseptique de la peau.

21. La composition la revendication 20, dans laquelle ladite composition comprend une teneur en actif comprise entre 1 et 20% du poids total de la composition.

22. La composition la revendication 20, dans laquelle ladite composition comprend une teneur en actif comprise entre 1 et 10% du poids total de la composition.

23. Une méthode de soin cosmétique et non-thérapeutique comprenant une étape d'application sur la peau d'un sujet d'une quantité efficace d'une composition selon l'une quelconque des revendications 1 à 20.

24. Une utilisation cosmétique et non-thérapeutique d'une composition selon l'une quelconque des revendications 1 à 22 pour opérer un traitement en profondeur qui stimule la microcirculation et la production d'énergie pour dynamiser les cellules et accélérer leur renouvellement, protéger le collagène et lutter contre les taches de pigmentation.

## Patentansprüche

1. Kosmetische oder dermatologische Zusammensetzung, **dadurch gekennzeichnet, dass** sie Folgendes umfasst:
(i) mindestens pflanzliche Öle reich an Omega-3-und/oder Omega-6-Fettsäuren, die aus der Gruppe, umfassend die Öle von Natternköpfen, Leindotter, Nachtkerze und Borretsch, ausgewählt werden, wobei diese pflanzlichen Öle in einem Verhältnis vorhanden sind, das es ermöglicht, ein Verhältnis von Omega-3-zu Omega-6-Fettsäuren in der Zusammensetzung zwischen 1 und 5 zu erhalten.
(ii) mindestens ein Mineral, vorzugsweise in Form von Salz, das aus der Gruppe, umfassend Natrium, Kalzium und Magnesium, ausgewählt wird,
(iii) mindestens ein Oligoelement, das aus der Gruppe, umfassend Zink, Kupfer und Eisen, ausgewählt ist,
(iv) mindestens Vitamin C oder ein Derivat desselben, und
(v) mindestens ein ätherisches Öl, das Radikalfänger-Eigenschaften hat, wobei das ätherische Öl ein ätherisches Öl von Strohblumen (Helichrysum Italicum) ist.

2. Zusammensetzung nach Anspruch 1, bei der die Zusammensetzung einen Gehalt an ätherischem Öl von Strohblumen zwischen 0,05 % und 5 % des Gesamtgewichts der Zusammensetzung umfasst.

3. Zusammensetzung nach Anspruch 1, bei der die Zusammensetzung einen Gehalt an ätherischem Öl von Strohblumen zwischen 0,05 % und 2,5 % des Gesamtgewichts der Zusammensetzung umfasst.

4. Zusammensetzung nach einem der vorhergehenden Ansprüche, bei der das Mineral Magnesium ist.

5. Zusammensetzung nach einem der vorhergehenden Ansprüche, bei der das mindestens eine Oligoelement aus der Gruppe, umfassend Zink und Kupfer, ausgewählt ist.

6. Zusammensetzung nach einem der vorhergehenden Ansprüche, bei der die Zusammensetzung Öle von Natternköpfen, Leindotter, Nachtkerze und Borretsch umfasst.

7. Zusammensetzung nach einem der vorhergehenden Ansprüche, bei der die Zusammensetzung folgenden Gehalt umfasst an:
a) Öl von Natternköpfen zwischen 0,1 % und 10 % des Gesamtgewichts der Zusammensetzung, und/oder
b) Öl von Leindotter zwischen 0,1 % und 10 % des Gesamtgewichts der Zusammensetzung, und/oder
c) Öl von Borretsch zwischen 0,1 % und 10 % des Gesamtgewichts der Zusammensetzung, und/oder
d) Nachtkerzenöl zwischen 0,1 % und 10 % des Gesamtgewichts der Zusammensetzung.

8. Zusammensetzung nach Anspruch 7, bei der die Zusammensetzung einen Gehalt an Öl von Natternköpfen zwischen 0,1 % und 5 % des Gesamtgewichts der Zusammensetzung umfasst.

9. Zusammensetzung nach Anspruch 7 oder 8, bei der die Zusammensetzung einen Gehalt von Öl von Leindotter zwischen 0,1 % und 5 % des Gesamtgewichts der Zusammensetzung umfasst.

10. Zusammensetzung nach einem der Ansprüche 7 bis 9, bei der die Zusammensetzung einen Gehalt an Öl von Borretsch zwischen 0,1 % und 5 % des Gesamtgewichts der Zusammensetzung umfasst.

11. Zusammensetzung nach einem der Ansprüche 7 bis 10, bei der die Zusammensetzung einen Gehalt an Nachtkerzenöl zwischen 0,1 % und 5 % des Gesamtgewichts der Zusammensetzung umfasst.

12. Zusammensetzung nach einem der vorhergehenden Ansprüche, bei der die Zusammensetzung folgenden Gehalt umfasst an:
a) Magnesium zwischen 0,001 % und 5 % des Gesamtgewichts der Zusammensetzung, und/oder
b) Kupfer zwischen 0,001 % und 5 % des Gesamtgewichts der Zusammensetzung, und/oder
c) Zink zwischen 0,001 % und 5 % des Gesamtgewichts der Zusammensetzung.

13. Zusammensetzung nach Anspruch 12, bei der die Zusammensetzung einen Gehalt an Magnesium zwischen 0,001 % und 1 % des Gesamtgewichts der Zusammensetzung umfasst.

14. Zusammensetzung nach Anspruch 12 oder 13, bei der die Zusammensetzung einen Gehalt an Kupfer zwischen 0,001 % und 1 % des Gesamtgewichts der Zusammensetzung umfasst.

15. Zusammensetzung nach einem der vorhergehenden Ansprüche, bei der die Zusammensetzung einen Gehalt an Zink zwischen 0,001 % und 1 % des Gesamtgewichts der Zusammensetzung umfasst.

16. Zusammensetzung nach einem der vorhergehenden Ansprüche, bei der die Zusammensetzung Zink, Kupfer und Magnesium umfasst.

17. Zusammensetzung nach einem der vorhergehenden Ansprüche, bei der die Zusammensetzung einen Gehalt an Vitamin C oder eines seiner Derivate zwischen 0,5 % und 10 % des Gesamtgewichts der Zusammensetzung umfasst.

18. Zusammensetzung nach Anspruch 17, bei der die Zusammensetzung einen Gehalt an Vitamin C oder eines seiner Derivate zwischen 0,5 % und 5 % des Gesamtgewichts der Zusammensetzung umfasst.

19. Zusammensetzung nach einem der vorhergehenden Ansprüche, bei der die Zusammensetzung ein Derivat von Vitamin C umfasst, vorzugsweise Ascorbinsäure 2-Glucosid (Ascorbic Acid 2-Glucoside oder 0-α-D-Glucopyranosyl-(1→2)-L-ascorbic acid).

20. Zusammensetzung nach einem der vorhergehenden Ansprüche, bei der die Zusammensetzung ferner mindestens ein weiteres Agens umfasst, das aus der Gruppe, umfassend die affinierenden, restrukturierenden, entzündungshemmenden, feuchtigkeitsspendenden Wirkstoffe für die Haut, ausgewählt ist.

21. Zusammensetzung nach Anspruch 20, bei der die Zusammensetzung einen Agens-Gehalt zwischen 1 % und 20 % des Gesamtgewichts der Zusammensetzung umfasst.

22. Zusammensetzung nach Anspruch 20, bei der die Zusammensetzung einen Agens-Gehalt zwischen 1 % und 10 % des Gesamtgewichts der Zusammensetzung umfasst.

23. Kosmetische und nicht therapeutische Pflegemethode, umfassend einen Schritt des Auftragens auf die Haut einer Person einer Wirkmenge einer Zusammensetzung nach einem der Ansprüche 1 bis 20.

24. Kosmetische und nicht therapeutische Verwendung einer Zusammensetzung nach einem der Ansprüche 1 bis 22, um eine Tiefenbehandlung durchzuführen, die die Mikrozirkulation und die Energieproduktion stimuliert, um die Zellen dynamisch zu machen und ihre Erneuerung zu beschleunigen, das Kollagen zu schützen und Pigmentflecken zu bekämpfen.

## Claims

1. A cosmetic or dermatological composition, **characterized in that** it comprises:
(i) at least plant oils which are rich in omega-3 and/or in omega-6, selected from the group comprising echium, camelina, evening primrose and borage oils, which plant oils are present in a proportion which makes it possible to obtain a ratio of omega-3 to omega-6 in said composition of between 1 and 5,
(ii) at least one mineral, selected from the group comprising sodium, calcium and magnesium,
(iii) at least one trace element selected from the group comprising zinc, copper and iron,
(iv) at least vitamin C or a derivative thereof, and
(v) at least one essential oil, which has anti-free radical properties, said essential oil is a Helichrysum essential oil (Helichrysum italicum).

2. The composition according to claim 1, wherein said composition comprises a content of Helichrysum essential oil of between 0.05% and 5% of the total weight of the composition.

3. The composition according to claim 1, wherein said composition comprises a content of Helichrysum essential oil of between 0.05% and 2,5% of the total weight of the composition.

4. The composition according any of the preceding claims, wherein the mineral is magnesium.

5. The composition according any of the preceding claims, wherein said at least one trace element is selected in the group comprising zinc and copper.

6. The composition according any of the preceding claims, wherein said composition comprises echium, camelina, evening primrose and borage oils

7. The composition according any of the preceding claims, wherein said composition comprises a content of:
a) echium oil between 0.1% and 10% of the total weight of the composition, and/or
b) camelina oil between 0.1% and 10% of the total weight of the composition, and/or
c) borage oil between 0.1% and 10% of the total weight of the composition, and/or
d) evening primrose oil between 0.1% and 10% of the total weight of the composition.

8. The composition according to claim 7, wherein said composition comprises a content of echium oil between 0.1% and 5% of the total weight of the composition.

9. The composition according to claim 7 or 8, wherein said composition comprises a content of camelina oil between 0.1% and 5% of the total weight of the composition.

10. The composition according to any of claims 7 to 9, wherein said composition comprises a content of borage oil between 0.1% and 5% of the total weight of the composition.

11. The composition according to any of claims 7 to 10, wherein said composition comprises a content of evening primrose between 0.1% and 5% of the total weight of the composition.

12. The composition according to any of the preceding, wherein said composition comprises a content of:
a) magnesium between 0.001% and 5% of the total weight of the composition, and/or
b) copper between 0.001% and 5% of the total weight of the composition, and/or
c) zinc of between 0.001% and 5% of the total weight of the composition.

13. The composition according to claim 12, wherein said composition comprises a content of magnesium between 0.001% and 1% of the total weight of the composition.

14. The composition according to claim 12 or 13, wherein said composition comprises a content of copper between 0.001% and 1% of the total weight of the composition.

15. The composition according to any of preceding claims, wherein said composition comprises a content of zinc between 0.001% and 1% of the total weight of the composition.

16. The composition according to any of preceding claims, wherein said composition comprises zinc, copper and magnesium.

17. The composition according to any of preceding claims, wherein said composition comprises a content of vitamin C or one of its derivatives of between 0.5% and 10% of the total weight of the composition.

18. The composition according to claim 17, wherein said composition comprises a content of vitamin C or one of its derivatives of between 0.5% and 5% of the total weight of the composition.

19. The composition according to any of the preceding claims, wherein said composition comprises a vitamin C derivative, preferably of ascorbic acid 2-glucoside (ascorbic acid 2-glucoside or 0-α-D-glucopyranosyl-(1→2)-L-ascorbic acid.

20. The composition according to any of the preceding claims, wherein said composition furthermore comprises at least one other active ingredient selected from the group comprising refining, restructuring, anti-inflammatory, hydrating and antiseptic agents for the skin.

21. The composition according to claim 20, wherein said composition comprises a content of said active ingredient between 1 and 20% of the total weight of the composition.

22. The composition according to claim 20, wherein said composition comprises a content of said active ingredient between 1 and 10% of the total weight of the composition.

23. A cosmetic care and non therapeutic method comprising a step of applying to the skin of a subject an effective quantity of a composition according to any claims 1 to 20.

24. A cosmetic and non therapeutic use of a composition according any of claims 1 to 22 for carrying out a deep treatment which stimulates the microcirculation and the production of energy in order to revitalise the cells and to accelerate their renewal, to protect the collagen and to fight against pigmentation marks.
